# EUROPÄISCHE PATENTSCHRIFT

(11) **EP 1 756 263 B1**
(45) Veröffentlichungstag und Bekanntmachung des Hinweises auf die Patenterteilung: **09.01.2008**
(21) Anmeldenummer: 04738699.0
(22) Anmeldetag: 14.06.2004
(51) Int. Cl.: C12M 3/06

(54) **FLÜSSIG-GAS-PHASENEXPOSITIONSREAKTOR FÜR DIE ZELLKULTIVIERUNG**
LIQUID/GAS PHASE EXPOSURE REACTOR FOR CELL CULTIVATION
REACTEUR D'EXPOSITION A UNE PHASE LIQUIDE-GAZEUSE POUR LA CULTURE DE CELLULES

(43) Veröffentlichungstag der Anmeldung: 28.02.2007
(73) Patentinhaber: ProBioGen AG, 13086 Berlin (DE)
(72) Erfinder: MARX, Uwe, 13129 Berlin (DE); RIEDEL, Marco, 12587 Berlin (DE); BUSHNAQ-JOSTING, Hikmat, 10439 Berlin (DE)
(74) Vertreter: Wehlan, Helmut
(86) Internationale Anmeldenummer: PCT/DE2004/001248
(87) Internationale Veröffentlichungsnummer: WO 2005/121311

(56) Entgegenhaltungen:
- WO-A-03/064586
- DE-A1- 10 211 106
- CHEMIE INGENIEUR TECHNIK, Nr. 12, 2003, Seiten 1888-1889, XP002329385

## Beschreibung

### Beschreibung

Die Erfindung betrifft ein Verfahren und eine Vorrichtung zur Anzucht von Zellen und zur Kultivierung von Zellen in hohen Dichten, wobei sich die zu kultivierenden Zellen in Hohlfadenmembranen befinden und abwechselnd in ein flüssiges Nährmedium und eine darüber befindliche Gasphase gebracht werden.

### Stand der Technik

Säugerzellkultivierung für die Herstellung von biopharmazeutischen Arzneimitteln wird überwiegend in Rührreaktoren betrieben. Weniger häufig kommen bisher Airliftreaktoren und sehr selten Hohlfaserreaktoren für die Marktbedienung mit säugerzeilbasierenden Arzneimitteln zum Einsatz. Zur Verbesserung der volumetrischen Produktausbeuten werden in den Rührreaktoren die Zelldichte und die effektive Produktionszeit der Zellen durch Medienoptimierung und zelllinienspezifische. Zufütterregime in sogenannten Fed-batch-Verfahren erhöht. Die Produktionstechnologie ist in Bioreaktorstrassen mit drei bis vier Rührkesseln ausgelegt, deren Fassungsvolumen jeweils etwa das Fünffache des vorhergehenden Bioreaktors betragen. Der größte verfügbare Rührreaktor für die Kultivierung von Säugerzellen hat gegenwärtig ein Fassungsvolumen von 20.000 Liter. Fed-batch-Prozesse in Rührreaktoren sind robust, im Maßstab bis zu den genannten Volumina vergrößerbar und für die Arzneimittelherstellung seit langem behördlich akzeptiert. Nachteile sind die langen Verweilzeiten der Produkte im Kulturraum, die Notwendigkeit der Zellabtrennung von dem Ernteüberstand, die durch die Mehrfachnutzung entstehenden Aufwendungen für Reinigung und Sterilisation sowie die hohen Investitions- und Betriebsaufwendungen für mit dieser Technologie ausgerüstete Werke.

Für degradationsanfällige Proteine, deren Herstellung eine geringe Verweilzeit im Bioreaktor voraussetzt, wie z.B. Faktor VII, wurden Vorrichtungen und Systeme entwickelt, die die Perfusion des Kulturraums und damit den kontinuierlichen Betrieb der Rührreaktoren erlauben. Hierfür ist der effiziente Zellrückhalt bei kontinuierlicher Medienzufuhr und Produkternte notwendig. Im Innenraum des Rührkessels kommen hier Spinfilter und in den Fällen, wo die Produktionszellen an Oberflächen anheften können, Trägermaterialen in Fließbett- oder Festbettverfahren zum Einsatz. Die kontinuierliche Betriebsweise von Rührkesseln kann auch über externe Zellrückhaltesysteme, wie z.B. Zellsedimentation, kontinuierliche Zellzentrifugen oder Ultraschallzellabscheider realisiert werden. Vorteile der kontinuierlichen Betriebsweise sind kurze Produktverweilzeiten im Bioreaktor, gleichbleibende Produktqualität während der Herstellung, Erhöhung der volumetrischen Produktivität und Flexibilisierung des Chargenvolumens über die festzulegende Kulturzeit Nachteile sind die Verunreinigung der Ernte mit Restzellen, die durch die Mehrfachnutzung entstehenden Aufwendungen für Reinigung und Sterilisation sowie die hohen Investitions- und Betriebskosten für die entsprechenden Werke.

Neben den Hohlfaserbioreaktoren der ACUSYST^{®} X Cell Generation, die sich für die Biopharmazeutika Herstellung bewährt haben, gibt es weitere Reaktorsysteme, in denen alle Zellkultur berührenden Komponenten als Einwegkomponenten ausgelegt sind. Diese können nach der Nutzung zur Herstellung einer Charge entsorgt werden. Aufwendige Reinigungs- und Sterilisationsprozeduren entfallen. Kommerziell erhältliche Systeme sind hier membranbasierende Systeme wie z.B. Cell-Pharm^{®}, Cellmax^{®}, Technomouse^{®}, CELLine^{®}, mini-PERM^{®} oder OptiCell^{®}. Membranverfahren besitzen mehrere Vorteile. Im Perfusionsbetrieb können sie - bedingt durch eine große Membranfläche pro Volumeneinheit- sehr hohe Zelldichten (10⁷-10⁸ Z/ml) erreichen. Außerdem sind die Zellen durch die Membranen vor Scherkräften geschützt. Grundsätzlich basieren sie auf dem Einwegprinzip, so dass Reinigung und Sterilisation nach Nutzung entfallen. Für die biopharmazeutische Herstellung hat sich im Bereich der Einwegbioreaktoren bisher noch der sogenannte Wave-Bioreaktor in den Erprobungsphasen bewährt. Die Zellen werden in dem System in einem zur Verbesserung der Durchmischung gezielt bewegten Beutelsystem kultiviert. Vorteil dieser Reaktortechnik ist die Einwegnutzbarkeit des Kultursystems. Nachteile sind die geringen erreichbaren Zelldichten und die limitierte Maßstabsvergrößerung.

In allen genannten Verfahren und Vorrichtungen ist die gleichmäßige Nährstoffversorgung und insbesondere die Sauerstoffversorgung bei hohen Zelldichten problematisch. Sowohl der Versuch, dieses Problem über komplexe Verfahrensschritte mit Druckbeaufschlagung zu lösen (1989, US 4,804,628 A), als auch das direkte Einbringen von Sauerstoff in den Zellkulturraum über ein weiteres Membransystem (1986, DE 24 31 450 A1 und 1995, DE 42 30194 A1) führten nicht zu beliebig im Maßstab vergrößerbaren Kultursystemen, in denen die Zellen gleichmäßig versorgt werden können. Bei Hohlfaserbioreaktoren, in denen die Zellen zwischen den Hohlfasern kultiviert und die Nährstoffe im Lumen der Faser transportiert werden, ist die Maßstabsvergrößerung durch die Länge der Hohlfasern limitiert. Die Länge der Hohlfasern ist jedoch durch den Verbrauch des Sauerstoffs aus den Hohlfasern begrenzt. Dadurch ist eine Maßstabsvergrößerung nur durch Parallelisierung möglich. Dies führt aber in der Praxis zu unrentablen Prozessen, d.h. die Skalierbarkeit der Hohlfaserbioreaktoren scheitert an einer adäquaten homogenen Versorgung der Zellen mit frischen Gas- und Flüssignährstoffkomponenten.

In der Patentanmeldung WO03/064586 A2 wurde vorgeschlagen, Zellen in hoher Dichte in Kammern zu kultivieren, wobei die Kammern in einer Dimension die Länge von 5 mm nicht überschreiten. Der Innenraum der Kammern bildet einen Kulturraum, der vom Versorgungsraum semipermeabel getrennt ist. Die Zellen werden in den Kammern zurückgehalten und der Stoffaustausch erfolgt über Hohlfasermembranen. Die Versorgung der Zellen mit Nährstoffen und mit Sauerstoff wird mittels eines variabel einstellbaren Gas/Zellkulturmediengemischs gewährleistet. Wenngleich Kulturvorrichtung und Verfahren die Probleme der Nährstoff und Sauerstoffversorgung lösen und Skalierbarkeit garantieren, so besteht ein Nachteil des in WO 03/064586 A2 beschriebenen Verfahrens darin, dass Zellen hoher Dichte in die Kammern eingebracht werden müssen. Zur Beseitigung dieses Nachteils wird in der Patentanmeldung WO 03/102123 A2 vorgeschlagen, zur Reduzierung der Animpfdichte im Beginn der Kultivierung von Zellen biodegradierbare Gele einzusetzen.

Ein Flüssig-Gas-Phasenexpositions-Bioreaktor wurde prinzipiell durch die Firma Zellwerk^{®} entwickelt und wird durch die Firma Sartorius^{®} vertrieben. In diesem Bioreaktor sind die auf Oberflächen anheftenden Zellen auf Scheiben aus Trägermaterial immobilisiert. Die Scheiben sind hintereinander auf einer Achse angeordnet und werden in einem zur Hälfte mit Medium zur Hälfte mit Gas gefüllten Zylinder rotiert. Vorteil dieser Anordnung ist die zyklische Exposition dieser Zellen in den beiden Phasen. Nachteile sind die Limitierung des Systems und Verfahrens auf anheftende Zellen, der Verbleib von abgelösten Zellen in der Ernteflüssigkeit sowie die Limitation bei der Maßstabsvergrößerung.

### Aufgabe der Erfindung

Die Aufgabe der Erfindung besteht darin, das in WO 03/064586 A2 beschriebene Verfahren weiter zu verbessern.

### Lösung der Aufgabe

Die Aufgabe wurde - wie in den Patentansprüchen formuliert - gelöst. Erfindungsgemäß wird die Anzucht und Kultivierung von Zellen in einem Flüssig-Gas-Phasenexpositions-Bioreaktor mit einem Versorgungsraum, in dem sich Hohlfadenmembranen mit einem Innendurchmesser von nicht mehr als 5 mm befinden und deren Innenvolumen Kulturkammern bilden, vorgenommen. Folgende Verfahrensschritte werden durchgeführt:
➢ Einbringen der Zellen in die Kulturkammern
➢ Befüllen etwa der Hälfte des Versorgungsraums mit Nährmedium, und der anderen Hälfte mit einem Gasgemisch
➢ gleichzeitiges oder versetztes Einschalten der Medium- und Gasperfusion
➢ zyklische Exposition der Hohlfadenmembranen und der darin befindlichen Zellen in der Gas- bzw. Flüssigphase

Gemäß einer bevorzugten Ausführungsvariante des erfindungsgemäßen Verfahrens sind die Hohlfadenmembranen waagerecht im Bioreaktor angeordnet. Nach dem Befüllen des Reaktors sind die Hälfte der Membranen mit Nährmedium bedeckt. Durch Drehung des Reaktors um 360° in eine Richtung und anschließend in die andere Richtung wird die zyklische Exposition der Hohlfadenmembranen und damit der Zellen in der Gas- bzw. Flüssigphase erreicht.

Die Drehung in die eine und anschließend in die andere Richtung verhindert ein Verdrillen der an den Reaktor angeschlossenen Schläuche.

Um jeweils gleiche Expositionszeiten in der Gas- bzw. Flüssigphase zu erreichen, wird erfindungsgemäß die Drehung nach 180° für eine bestimmte Zeit unterbrochen. Die Standzeiten sind variabel einstellbar. Dadurch wird gewährleistet, dass die Zellen während des Aufenthalts der Membranen im flüssigen Nährmedium ausreichend mit Nährstoffen und während des Aufenthalts in der Gasphase ausreichend mit Sauerstoff versorgt werden. Gleichzeitig kann durch Variation der Standzeiten den individuellen Stoffwechselbedürfnissen der einzelnen Zelllinien Rechnung getragen werden.

Alternativ dazu kann die zyklische Exposition der Hohlfadenmembranen durch Tauchen der Hohlfadenmembranen in das Nährmedium und anschließendes Herausheben in die Gasphase erfolgen. Durch diese Vorgehensweise können unterschiedliche Verweilzeiten der Zellen in den beiden Phasen erreicht werden.

Zur Durchführung des erfindungsgemäßen Verfahrens werden zunächst Zellen geringer Dichte in den Kulturraum eingebracht und wachsen dann zu Zellen hoher Dichte heran. Durch den Einsatz von Gelen- wie in der Patentanmeldung WO 03/102123 A2 beschriebenist es möglich, Zellen geringster Zelldichte zusammen mit Gelen aus vernetzten Polypeptiden, die einem hohen Glutaminanteil aufweisen und / oder mit semisoliden Medien aus viskose Flüssigkeiten oder Flüssigkeiten aus mikroskopisch kleinen Gelstücken in den Kulturraum einzubringen.

Das Einbringen der Zellen in die Kammern erfolgt über ein zentrales Beschickungssystem außerhalb des Versorgungsraums, so dass der gleichzeitige gleichmäßige Eintrag der Zellen in alle Kammern über einen Anschluss möglich ist.

Das erfindungsgemäße Verfahren eignet sich, um Protozoen, Bakterien, Hefen, Pilze, Pflanzen- oder Säugerzellen zu kultivieren.

Das Verfahren ist gegenüber dem in der Patentanmeldung WO 03/064586 A2 beschriebenen Verfahren neu, da dort keine Exposition der Zellen in zwei verschiedenen Phasen, sondern nur die Exposition der Zellen in einem variabel einstellbaren Gas/Zellkulturmediengemisch vorgesehen ist. Auch ist dort keine Bewegung des Reaktors oder der Membranen beschrieben worden. Man erhält aus WO 03/064586 A2 auch keinen Hinweis darauf, die Membranen mit den Zellen bestimmte Zeiten in den entsprechenden Phasen zu bewegen. Dies liegt daran, dass in der genannten Druckschrift eine Einrichtung zur Erzeugung des variabel einstellbaren Gas/Zellkulturmediengemisches benötigt wird und somit eine Bewegung der Membranen- vor allem eine Rotation- weitere komplizierte Maßnahmen hinsichtlich der Anschlüsse erforderlich machen würden.

Die erfindungsgemäße Vorrichtung besteht aus einem zylinder- oder kugelförmigen (mit Gas bzw. Medium beschickbaren) Zweiphasen-Versorgungsraum, in dem - parallel zur Längsachse des Zylindermantels - polymere zellrückhaltende Mikrofiltrations- Hohlfadenmembranen mit einem Innendurchmesser von nicht mehr als 5 mm in den Endplatten fixiert sind, deren Innenvolumen Kulturkammern bilden, in denen sich die zu kultivierenden Zellen befinden, wobei der Versorgungsraum eine mit Gasgemisch durchströmbare Gasphase und eine mit Kulturmedium durchströmbare Flüssigkeitsphase enthält, jede Hohlfadenmembran über die Zylinderlänge einen Abstand von mindestens 0.5 mm zu der nächstliegenden Hohlfadenmembran hat, die Hohlfadenmembranen bezüglich eines gedachten Querschnitts entlang der Rotationsachse des Zylinders symmetrisch angeordnet sind und sich auf einer gedachten Querschnittsebene entlang der Rotationsachse des Zylinders keine Membranen befinden.

Die Membranen sind für alle Stoffe, jedoch nicht für ganze Zellen durchlässig. Der Kulturraum, bestehend aus dem Gesamtvolumen der Kammern, ist vom Versorgungsraum durch die Membran getrennt. Diese erlaubt den Versorgungssubstraten das Eintreten in den Kulturraum und die Versorgung der Zellen. Das Trennsystem erlaubt auch das Austreten von Produkten aus den Zellkammern in die Versorgungsumgebung.

Die Membranen bestehen aus Polymeren, z.B. aus Polysulfon, Polyethersulfon oder Polycarbonat. Als besonders geeignet haben sich Hohlfadenmembranen aus dem biokompatiblen Material Polyethersulfon erwiesen, die Membranwandstärken kleiner 300 µm, Wasserpermeabilität größer 6 m³/m²*h*Bar und Porendurchmesser von 0,1 bis 1,0 µm aufweisen.

Zur Verhinderung der Ausbildung von Flüssigkeitsfilmen und damit zur gleichmäßigen Exposition der Membranen in der Gasphase haben die Membranen einen Mindestabstand untereinander.

Die Membranen sind vorzugsweise hexagonal angeordnet. Das bedeutet, dass jede Membran - mit Ausnahme derer, die sich an den äußeren Rändern befinden - von je 6 Membranen mit gleichem Abstand zu der zentralen Membran umgeben sind. Damit kann die höchste gleichmäßige Packungsdichte im Versorgungsraum gewährleistet werden. Weitere platzbeanspruchende Einrichtungen sind nicht notwendig.

Die Hohlfadenmembranen sind bezüglich eines gedachten Querschnitts entlang der Rotationsachse des Zylinders - welcher praktisch die Phasengrenze darstellt - symmetrisch angeordnet. Auf der gedachten Querschnittsebene entlang der Rotationsachse des Zylinders befinden sich keine Membranen. Damit wird sichergestellt, dass während der Standzeiten alle Membranen sich entweder vollständig in der Gas- oder vollständig in der Flüssigphase befinden.

Die erfindungsgemäße Vorrichtung ist gegenüber der in WO 03/064586 A2 beschriebenen Vorrichtung neu, da dort kein Zwei-Phasen-Betrieb vorgesehen ist. Sie benötigt des Weiteren keine besondere Einrichtung für die Erzeugung eines Gas/ Mediengemisches sowie für die Flüssigkeitsabscheidung aus dem verbrauchten Gas/ Mediengemisch.

Jede Endplatte des Zylinders enthält mindestens zwei Anschlüsse für den Gasein- bzw. -austrag, die sich jeweils ober- und unterhalb der gedachten Querschnittsebene befinden, sodass die Gasversorgung kontinuierlich auch bei Drehung des Zylinders um seine Rotationsachse gewährleistet ist.

Weiterhin befindet sich an den Kopfflächen mindestens je ein Schlauchanschluss zur Medienperfusion und mindestens je ein Zugang zum Kulturraum für die Zelleinsaat.

Zusätzlich sind Schläuche, Gasbefeuchter, eine Mediumfalle in der Gasstrecke, eine Ultrafiltrations-Einheit in einer Produkterntestrecke, eine Hardware-Einheit, Pumpen, Mess- und Regeleinheiten sowie ein Antriebsmotor und ein Gestell, welches die Lagerung und Rotation der Vorrichtung ermöglicht, enthalten.

Die Ultrafiltrations-Einheit in einer Produkterntestrecke dient der Konzentration des jeweiligen Produkts.

Überraschenderweise hat sich herausgestellt, dass mit der erfindungsgemäßen Vorrichtung eine höhere Zelldichte und damit eine höhere Ausbeute an Zellprodukten als mit der Vorrichtung gemäß WO 03/064586 A2 erzielt werden konnte. Dies lässt sich einerseits auf die optimale Platzausnutzung und andererseits auf die verbesserte Versorgung der Zellen durch zyklische Exposition der Hohlfadenmembranen in den zwei Phasen zurückführen.

Alternativ dazu können die Anschlüsse für die Gasversorgung nicht an den Kopfflächen, sondern am Zylindermantel, ober- und unterhalb der gedachten Querschnittsebene, angebracht sein.

Die erfindungsgemäße Verwendung der Vorrichtung liegt in der Kultivierung von Zellen in hohen Dichten und in der Gewinnung von Zellprodukten, Zellbestandteilen, Viren, Proteinen oder niedermolekularen Substanzen, z.B. von Arzneimitteln sowie Diagnostika- und Forschungsreagenzien.

Die Merkmale der Erfindung gehen aus den Elementen der Ansprüche und aus der Beschreibung hervor, wobei sowohl einzelne Merkmale als auch mehrere in Form von Kombinationen vorteilhafte Ausführungen darstellen, für die mit dieser Schrift Schutz beantragt wird.

Das Wesen der Erfindung besteht aus einer Kombination aus bekannten (Trennung von Kultur- und Versorgungsraum durch Membranen) und neuen Elementen (Anordnung der Membranen im Versorgungsraum, Drehbarkeit der Vorrichtung, wechselnde Exposition der Zellen in der Gas- bzw. Flüssigkeitsphase), die sich gegenseitig beeinflussen und in ihrer neuen Gesamtwirkung einen Gebrauchsvorteil und den erstrebten Erfolg ergeben, der darin liegt, dass eine Möglichkeit zur effektiven kontinuierlichen Kultivierung von Zellen in hoher Dichte und zur Produktgewinnung aus diesen Zellen bei gleichzeitigem Zellrückhalt bereitgestellt wird.

Im Folgenden soll die Erfindung und deren Funktion anhand von Figuren erläutert werden. Die Figuren dienen nur dem besseren Verständnis, sie sollen jedoch nicht als einzige Bauweise verstanden werden, mit der die Ansprüche realisiert werden können.

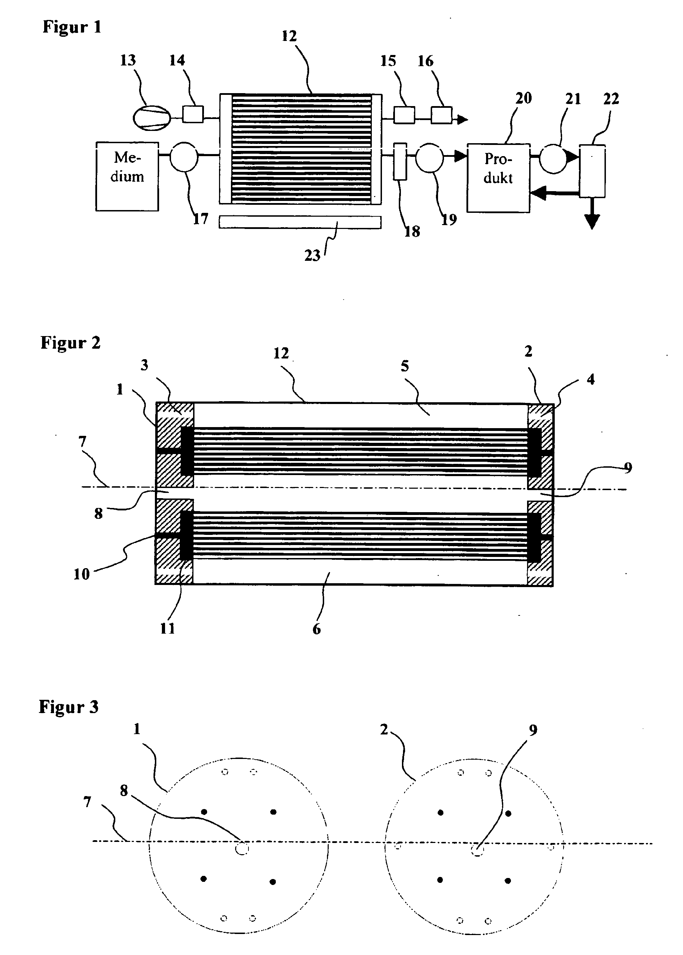

### Figur 1: Schema des Bioreaktorsystems

Die Figur stellt eine Variante des Bioreaktorsystems auf der Basis eines zylindrischen Versorgungsraumes (12) dar. Die Ausrichtung, nicht aber die realen Dimensionen und die tatsächliche Anzahl der im Versorgungsraum angeordneten Hohlfadenkulturkammern, ist durch die schwarzen Linien im Versorgungsraum dargestellt. Das zylindrische Gefäß wird von einer Drehvorrichtung (23) im geeigneten Rhythmus periodisch richtungswechselnd bewegt. Die Durchströmung der Gasphase des Versorgungsraumes wird durch eine Gasstrecke sichergestellt, die aus einer Gasmischstation(13) und einem Gasbefeuchter(14) für die Gaszufuhr in den Zylinder und aus einer Medienfalle (15) und einer Kontaminationsfalle (16) für die Gasabfuhr aus dem Zylinder besteht. Die Durchströmung der Flüssigphase des Zylinders wird durch eine Medienstrecke sichergestellt, die aus dem Medienreservoire und einer Pumpe (17) für die Medienzufuhr sowie einer Pumpe (19) und dem Produktsammelgefäß (20) für die Abfuhr aus dem Zylinder besteht. In der Abfuhrstrecke ist des Weiteren eine Messsondenstrasse(18) für die Vermessung von Sauerstoff, pH-Wert und Temperatur integriert. An das Produktsammelgefäß ist zur Konzentration des Produktes im Produktsammelgefäß (20) ein Kreislauf mit einer Pumpe (21) und einem Ultrafiltrationsmodul (22) angeschlossen. Das produktfreie Filtrat wird aus diesem Ultrafiltrationsmodul nach unten abgeführt und verworfen, währenddessen das konzentrierte Produkt in das Produktsammelgefäß zurückgeführt wird. Vorteilhafterweise sind alle Elemente des Bioreaktorsystems beginnend mit dem Anschluss an die Gasmischstation Einwegmaterialien. Die Pumpen sind als Schlauchpumpen ausgelegt.

### Figur 2: Längsschnitt des zylindrischen Zweiphasenversorgungsraums

Der zylindrische Versorgungsraum (12) enthält während des Reaktorbetriebes im oberen Bereich eine Gasphase (5) und im unteren Bereich eine Flüssigkeitsphase (6), die eine Phasengrenzfläche bilden (7). Links wird der Versorgungsraum durch eine Endplatte (1) für die Zufuhr von Gas und Medium und rechts durch eine Endplatte (2) für die Abfuhr begrenzt. Rechts begrenzt die begrenzt. Gas wird durch Ports (3, 4) in den Endplatten geführt. Der Medientransport erfolgt über jeweils einen auf der Zylinderrotationsachse gelegenen zentralen Port (8, 9) in den Endplatten. Die einzelnen Kulturkammern für die Zellen sind als gleiche Hohlfadenmembranen ausgelegt und im Versorgungsraum als parallele schwarze Linien dargestellt. Der Eintrag der Zellsuspension erfolgt getrennt von der Gas- und Medienversorgung durch die schwarz dargestellten Ports (10) in den Endplatten. Zur gleichmäßigen Einsaat der Zellen in alle Hohlfadenmembranen enden die Ports in Zellverteilerräumen (11), die mit dem Innenraum jeder einzelnen Hohlfadenmembran verbunden sind.

### Figur 3: Draufsicht der Endplatten des Versorgungsraums

In der Draufsicht der Zufuhrendplatte (1) ist eine der eingesetzten Anordnungen für die Zugänge für Gas - als kleine Kreise dargestellt - und für Medium (8) in den Versorgungsraum abgebildet. Die Draufsicht der Abfuhrendplatte (2) zeigt den zentralen Port für die Produktabfuhr (9) und eine eingesetzte Anordnung von Ports für die Gasabfuhr, die mit kleinen Kreisen dargestellt sind. In diesem Beispiel sind je vier durch schwarze Punkte dargestellte Ports für die Einsaat der Zellsuspension in die beiden Endplatten integriert, die über einen zusammengeführten Schlauchanschluss beschickt werden können.

Die Erfindung soll anhand von Ausführungsbeispielen erläutert werden, ohne auf diese Beispiele beschränkt zu sein.

### Ausführungsbeispiele:

### Beispiel 1: Zellen hoher Dichte

Zwei Bioreaktorsysteme wurde nach dem in Figur 1 abgebildeten Schema aufgebaut. Der zylindrische Versorgungsraum hatte ein Gesamtfassungsvolumen von 14 Litern. Die Flüssigkeitsphase beinhaltete im Prozess 7 Liter. In beiden Fällen wurden 144 Hohlfadenmembranen von jeweils 500 mm Länge axialsymmetrisch im Versorgungsraum angeordnet. Die Zelleinsaat erfolgte mit Zellen in hoher Dichte in dem Basismedium PBG1.0 unter Zusatz von 0.02 % Humanserumalbumin über die Einsaatports in den Endplatten in die Innenräume der Hohlfadenmembranen. Die Zelllinie produziert ein menschliches Protein, welches durch ein Einschrittchromatographieverfahren aus dem Kulturüberstand isoliert und dann exakt in seiner Menge bestimmt werden kann. Die Kulturzeit betrug 10 und 23 Tage. Über diese Zeit wurde die Gasphase des Versorgungsraumes kontinuierlich mit einem Gemisch von Luft und 5% CO₂ durchströmt. Insgesamt wurden entsprechend 11 Liter in 10 Tagen bzw. 24 Liter Kulturmedium in 23 Tagen für die Zellversorgung in den Läufen eingesetzt. Die Flüssig- bzw. Gasphasenexpositionszyklen betrugen im Experiment jeweils 30 Sekunden zwischen den Phasenwechseln. Nach Abschluss der Kulturführung wurden die Zellen aus den Hohlfadenmembranen über die Einsaatports geerntet und Zelldichte und Vitalität bestimmt. Das Protein wurde ans einem Aliquot der zellfreien Produkternte isoliert und in der Menge bestimmt. In nachfolgender Tabelle ist die in den Hohlfadenkulturkammern erreichte Zelldichte und Vitalität aufgeführt.

| | Bioreaktorlauf 1 (10Tage) | Bioreaktorlauf 2 (23 Tage) |
|---|---|---|
| Gesamtzellzahl Inokulum [Zellen pro ml Kulturraum] | 1,5E7 | 1,8E7 |
| Vitalität Inokulum [%] | 67 | 80 |
| Gesamtzellzahl Ernte | 2,4E7 | 2,25E7 |
| Vitalität Ernte (%) | 54 | 29 |
| Gesamtproteinmenge (mg) | 48 | 168 |

Im System konnten hohe Zelldichten erhalten werden. Darüber hinaus wurden während des Prozesses 48mg und 168mg Protein gebildet und in dem zellfreien Kulturüberstand in dem entsprechenden Produktsammelgefäß aufgefangen.

### Beispiel 2: Zellen geringer Dichte

Zellen in Lebendzelldichten von 1,3E5 Zellen pro Milliliter Kulturraum wurden in zwei Zweiphasenexpositionsreaktoren mit je 12 Hohlfadenmembranen von einer Länge von 200mm inokuliert und über 4 Tage unter Drehung der beiden Kunststoffkessel kultiviert. Vor dem Inokulum wurden die Zellen mit mikroskopisch kleinen Gelstücken aus HSA gemischt. Der Medienaustausch erfolgte diskontinuierlich. Die Stoffwechselaktivität wurde über den Glukoseverbrauch vermessen. Die Zelldichten wurden nach Abschluss der Läufe durch Ernte des Gels mit den darin befindlichen Zellen und Auszählung über Trypanblau bestimmt. Es konnte innerhalb der kurzen Kulturzeit eine Expansion der Zellen auf 6E5 lebende Zellen pro Milliliter Kulturraum (4,6 fach) bzw. 5E5 lebende Zellen pro Milliliter Kulturraum (3,8 fach) erreicht werden.

### Beispiel 3: Funktionsweise des Bioreaktorsystems

Das Versorgungsprinzip des Bioreaktors basiert darauf, dass sich die Zellen abwechselnd in Medium und in einem Gasgemisch aufhalten und somit die Versorgung der Zellen mit Sauerstoff gegenüber herkömmlichen Systemen verbessert werden konnte.

### Beispiel 4: Aufbau des Systems

Das Herzstück des Bioreaktors bildet ein zylindrischer Kunststoffkessel, der waagerecht gelagert wird. In diesem Gefäß sind, parallel zur Rotationsachse, Hohlfadenmembranen über die Länge gespannt, wie in Figur 2 dargestellt. Hierdurch ergeben sich im Zylinder zwei voneinander durch die Membranen getrennte Räume. Dies ist zum Einen der die Hohlfadenmembranen umgebende Versorgungsraum, der aus einer Flüssigphase und einer Gasphase besteht. Die Grenzfläche zwischen diesen beiden Phasen ist in den Figuren 2 und 3 skizziert. Zum Anderen ist es der Raum innerhalb einer jeden Hohlfadenmembran. Die Summe aller Hohlfadeninnenräume stellt den Kulturraum für die Zellen dar. Über die Anzahl der rotationsachsensymmetrisch angeordneten Hohlfadenmembranen ist das system beliebig skalierbar bezüglich seines Kulturraumes. Beide Räume verfügen über separate Zugänge und sind voneinander getrennt. Die einzige Verbindung zwischen dem Versorgungsraum und dem Kulturraum stellen die Poren der Membran dar. Diese Poren sind mit einem vorteilhaften Porendurchmesser von 0,1 bis 1,0 µg pro Milliliter für kleine Moleküle sowie für Proteine durchgängig ist, nicht aber für die Zellen. Die kontinuierliche Durchströmbarkeit des Systems mit Gas bzw. Flüssigkeit wird durch die in Figur 1 aufgeführte und beschriebene Gesamtvorrichtung sichergestellt.

Zum Bioreaktorsystem gehört weiterhin eine mobile Hardware-Einheit, die Pumpen, Kompressoren sowie Mess- und Regeleinheiten beinhaltet. Weiterhin umfasst diese Einheit einen Antriebsmotor und eine Drehvorrichtung, die Lagerung und Rotation des Kunststoffkessels ermöglicht.

### Beispiel 5: Funktionsweise

Der Kunststoffkessel wird durch die Drehvorrichtung in einem auf die jeweilige Zelllinie anpassbaren Rotationszyklus um seine Rotationsachse gedreht. Dieser Rotationszyklus wird vorteilhafter weise über die gesamte Bioreaktorlaufzeit ununterbrochen wiederholt. Die acht Phasen eines Rotationszyklus sind nachfolgend beispielhaft aufgeführt.
Phase: Rechtsdrehung um 180°
Phase: Standzeit
Phase: Rechtsdrehung um 180°
Phase: Standzeit
Phase: Linksdrehung um 180°
Phase Standzeit
Phase: Linksdrehung um 180°
Phase: Standzeit

Im Ergebnis eines Rotationszyklus hat der Kessel eine volle Umdrehung in eine Richtung und eine volle Umdrehung in die entgegengesetzte Richtung absolviert und befindet sich wieder in der ursprünglichen Ausgangsposition. Der Wechsel der Drehrichtung erlaubt die Medien- und Gasdurchströmung über fest im Kessel integrierte Ports, an denen Kunststoffschläuche fixiert sind. Damit kommt das System anders als das Gros der Säugerzellbioreaktoren ohne bewegliche, in den sterilen Versorgungs- bzw. Kulturbereich hineinführende Baukomponenten, wie Impellerachsen oder Medien- bzw. Gaszufuhrrohre aus. Das damit verbundene Kontaminationsrisiko tritt also nicht auf und es entstehen keine Aufwendungen für die Sicherung entsprechender Reibungsflächen, durch z.B. doppelte Gleitringdichtungen.

Systemaufbau und Betriebsweise sichern gleichzeitig ab, dass unabhängig von der Anzahl der im System befindlichen Hohlfadenmembranen, jede einzelne über die gesamte Bioreaktorlaufzeit identischen Expositionsbedingungen in beiden Phasen ausgesetzt ist. Die Exposition in der Gasphase dient vor allem der Sauerstoffversorgung währenddessen die Exposition in der Flüssigphase vor allem der Aufnahme gelöster Nährstoffe und der Abfuhr von Stoffwechselprodukten dient. Sowohl Nährmedium als auch das Gasgemisch können kontinuierlicher zugeführt werden.

### Vorteile/ Neuheiten:

Kessel mit integriertem Zellrückhaltesystem als Einwegartikel
Membran ist für Protein durchlässig à zellfreie Ernte
Gleiche Expositionsbedingungen für jede einzelne Hohlfadenmembran im System kurze Diffusionswege für Sauerstoff bei Exposition in der Gasphase keine Gradientenbildung in der Gasphase des Expositionsreaktors über die Länge des Reaktors
Mehrere, entsprechend über die Endkappen verteilte Gasports, die kontinuierliche Gasdurchströmung auch während der Rotation ermöglichen

### Definitionen:

Zellen hoher Dichte: Eine Kultur mit hoher Zelldichte ist bei Zelldichten größer als 1E7 Zellen pro Milliliter Kulturraum erreicht.

Zellen geringer Dichte sind erreicht, wenn die Zelldichte im Kulturraum zwischen 1E4 und 1 E7 Zellen pro Milliliter Kulturraum liegt.

Zellen geringster Dichte: Eine Kultur mit geringster Zelldichte ist bei Dichten kleiner 1E4 Zellen pro Milliliter Kulturraum erreicht.

Nährmedium: Nährmedium ist eine wässrige Lösung, in der für die Zellen essentielle Nährstoffe wie z.B. Glucose, Aminosäuren und Spurenelemente enthalten sind.

Gasgemisch: Ein Gasgemisch im hier verwendeten Sinne beschreibt vorteilhafter Weise eine Mischung aus Luft und Kohlendioxid mit variablem Mischungsverhältnis. Weiterhin schließt der Begriff Gasgemisch in der vorliegenden Bedeutung auch variable Mischungsverhältnisse von Stickstoff, Sauerstoff und CO₂ ein.

### Bezugszeichenliste:

- 1: Zufuhrendplatte
- 2: Abfuhrendplatte
- 3, 4: Ports für die Gasver- und Entsorgung
- 5: Gasphase
- 6: Flüssigkeitsphase
- 7: Phasengrenzfläche
- 8: Zugang für Medium (zentraler Port)
- 9: zentraler Port für die Produktabfuhr
- 10: Ports für den Kulturraum
- 11: Zellverteilerräume
- 12: zylindrischer Versorgungsraum
- 13: Gasmischstation
- 14: Gasbefeuchter
- 15: Medienfalle
- 16: Kontaminationsfalle
- 17, 19, 21: Pumpen
- 22: Ultrafiltrationsmodul
- 23: Drehvorrichtung

## Patentansprüche

1. Verfahren zur Anzucht und Kultivierung von Zellen in einem Flüssig-Gas-Phasenexpositions-Bioreaktor mit einem Versorgungsraum, in dem sich Hohlfadenmembranen mit einem Innendurchmesser von nicht mehr als 5 mm befinden und deren Innenvolumen Kulturkammern bilden, **gekennzeichnet durch** folgende Schritte:
- Einbringen der Zellen in die Kulturkammern
- Befüllen etwa der Hälfte des Versorgungsraums mit Nährmedium, und der anderen Hälfte mit einem Gasgemisch
- gleichzeitiges oder versetztes Einschalten der Medium- und Gasperfusion
- zyklische Exposition der Hohlfadenmembranen und der darin befindlichen Zellen in der Gas- bzw. Flüssigphase

2. Verfahren nach Anspruch 1, **dadurch gekennzeichnet, dass** die Hohlfadenmembranen waagerecht angeordnet sind, nach dem Befüllen des Reaktors die Hälfte der Membranen mit Nährmedium bedeckt sind und die zyklische Exposition der Hohlfadenmembranen durch Drehung des Reaktors um 360° in eine Richtung und anschließend in die andere Richtung erreicht wird.

3. Verfahren nach Anspruch 2, **dadurch gekennzeichnet, dass** die Rotation in zwei 180°-Schritten erfolgt, die durch variabel einstellbare Wartezeiten voneinander getrennt werden, so dass sich die jede einzelne Hohlfasermembran die gleiche Zeit in der flüssigen Phase wie in der Gasphase befindet.

4. Verfahren nach Anspruch 1, **dadurch gekennzeichnet, dass** die zyklische Exposition durch Tauchen der Hohlfadenmembranen in das Nährmedium und anschließendes Herausheben in die Gasphase erreicht wird.

5. Verfahren nach Anspruch 1, **dadurch gekennzeichnet, dass** Zellen geringer Dichte in den Kulturraum eingebracht werden und zu Zellen hoher Dichte heranwachsen.

6. Verfahren nach Anspruch 5, **dadurch gekennzeichnet, dass** das Zellen geringster Zelldichte zusammen mit Gelen aus vernetzten Polypeptiden, die einem hohen Glutaminanteil aufweisen und / oder mit semisoliden Medien aus viskose Flüssigkeiten oder Flüssigkeiten aus mikroskopisch kleinen Gelstücken in den Kulturraum eingebracht werden.

7. Verfahren nach Anspruch 1, **dadurch gekennzeichnet, dass** es sich bei den Zellen um Protozoen, Bakterien, Hefen, Pilze, Pflanzen- oder Säugerzellen handelt.

8. Verfahren nach Anspruch 1, **dadurch gekennzeichnet, dass** das Einbringen der Zellen in die Kammern über ein zentrales Beschickungssystem außerhalb des Versorgungsraums erfolgt und dass der gleichzeitige {homogene} Eintrag der Zellen in alle Kammern über einen Anschluss möglich ist.

9. Vorrichtung zur Anzucht und Kultivierung von Zellen in einem zylinder- oder kugelförmigen (mit Gas bzw. Medium beschickbaren) Zweiphasen-Versorgungsraum, in dem - parallel zur Längsachse des Zylindermantels - polymere zellrückhaltende Mikrofiltrations- Hohlfadenmembranen mit einem Innendurchmesser von nicht mehr als 5 mm in den Endplatten fixiert sind, deren Innenvolumen Kulturkammern bilden, in denen sich die zu kultivierenden Zellen befinden, **dadurch gekennzeichnet, dass**
- der Versorgungsraum eine mit Gasgemisch durchströmbare Gasphase und eine mit Kulturmedium durchströmbare Flüssigkeitsphase enthält
- jede Hohlfadenmembran über die Zylinderlänge einen Abstand von mindestens 0,5mm zu der nächstliegenden Hohlfadenmembran hat
- die Hohlfadenmembranen bezüglich eines gedachten Querschnitts entlang der Längsachse des Zylinders symmetrisch angeordnet sind
- sich auf der gedachten Querschnittsebene entlang der Längsachse des Zylinders keine Membranen befinden

10. Vorrichtung nach Anspruch 9, **dadurch gekennzeichnet, dass** jede Endplatte des Zylinders mindestens zwei Anschlüsse für den Gasein- bzw. -austrag aufweist, die sich jeweils ober- und unterhalb der gedachten Querschnittsebene befinden und so der Versorgungsraum mit den Hohlfadenmembranen bei Medien- und Gasversorgung um seine Längsachse drehbar ist.

11. Vorrichtung nach Anspruch 9 und 10, **dadurch gekennzeichnet, dass** die Anschlüsse für die Gasversorgung nicht an den Kopfflächen, sondern am Zylindermantel, ober- und unterhalb der gedachten Querschnittsebene, angebracht sind.

12. Vorrichtung nach Anspruch 9, **dadurch gekennzeichnet, dass** die Hohlfadenmembranen eine Wandstärke von weniger als 300 µm aufweisen, eine Wasserpermeabilität größer 6m³/m²*h*Bar erreichen und einen Porendurchmesser von 0,1 bis 1 µm haben.

13. Vorrichtung nach Anspruch 9, **dadurch gekennzeichnet, dass** die Membranen im Versorgungsraum hexagonal angeordnet sind.

14. Vorrichtung nach Anspruch 9, **dadurch gekennzeichnet, dass** sich an den Kopfflächen mindestens je ein Schlauchanschluss zur Mediumperfusion und mindestens je ein Zugang zum Kulturraum befindet.

15. Vorrichtung nach mindestens einem der Ansprüche 10 bis 14, **dadurch gekennzeichnet, dass** sie zusätzlich Schläuche, Gasbefeuchter, eine Mediumfalle in der Gasstrecke, eine Ultrafiltrations-Einheit in einer Produkterntestrecke, eine Hardware-Einheit, Pumpen, Kompressoren, Mess- und Regeleinheiten sowie einen Antriebsmotor und ein Gestell, welches die Lagerung und Rotation der Vorrichtung ermöglicht, enthält.

16. Verwendung der Vorrichtung nach den Ansprüchen 9 bis 15 zur Kultivierung von Zellen in hohen Dichten und zur Gewinnung von Zellprodukten, Zellbestandteilen, Viren, Proteinen oder niedermolekularen Substanzen.

17. Verwendung nach den Anspruch 16 zur Gewinnung von Arzneimitteln.

18. Verwendung nach den Ansprüchen 16 und 17 zur Herstellung von Diagnostika.

## Claims

1. A method for initiation of growth and cultivation of cells in a liquid-gas-phase exposure bioreactor containing a supply chamber in which there are disposed hollow-filament membranes having an inside diameter of no larger than 5 mill and whose inner volume forms culture compartments, **characterized by** the following steps:
• introduction of the cells into the culture compartments
• filling approximately one half of the supply chamber with nutrient medium and the other half with a gas mixture
• turning on perfusion of medium and gas simultaneously or separately
• cyclic exposure of the hollow-filament membranes and of the cells contained therein in the gas or liquid phase

2. A method according to claim 1, **characterized in that** the hollow-filament membranes are oriented horizontally, after the reactor has been filled, half of the membranes are covered with nutrient medium, and cyclic exposure of the hollow-filament membranes is achieved by rotating the reactor 360° in one direction and then in the opposite direction.

3. A method according to claim 2, **characterized in that** the rotation takes place in two 180° steps, which are separated from one another by variably adjustable waiting times, so that each individual hollow-fiber membrane spends the same time in the liquid phase as in the gas phase.

4. A method according to claim 1, **characterized in that** the cyclic exposure is achieved by immersing the hollow-filament membranes in the nutrient medium and then lifting them into the gas phase.

5. A method according to claim 1, **characterized in that** cells of low density are introduced into the culture chamber and grow to cells of high density.

6. A method according to claim 5, **characterized in that** cells of the lowest cell density are introduced into the culture chamber together with gels of cross-linked polypeptides, which have a high glutamine content, and/or with semisolid media of viscous fluids or fluids composed of microscopically small gel fragments.

7. A method according to claim 1, **characterized in that** the cells are protozoa, bacteria, yeasts, fungi and plant or mammalian cells.

8. A method according to claim 1, **characterized in that** the cells are introduced into the compartments via a central charging system outside the supply chamber and **in that** simultaneous (homogeneous) input of the cells into all compartments is possible via one port.

9. A device for initiation of growth and cultivation of cells in a cylindrical or spherical two-phase supply chamber (which can be charged with gas and medium respectively), in which parallel to the longitudinal axis of the cylinder shell -polymeric, cell-retaining, micro filtering, hollow-filament membranes having an inside diameter of no more than 5 mm are fixed in the end plates, the inner volumes of which form culture compartments, in which the cells to be cultivated are disposed, **characterized in that**
• the supply chamber contains a gas phase through which a gas mixture can flow and a liquid phase through which a culture medium can flow
• each hollow-filament membrane has a spacing of at least 0.5 mm to the neighbouring hollow-filament membrane over the length of the cylinder
• the hollow-filament membranes are symmetrically disposed relative to an imaginary cross section along the longitudinal axis of the cylinder
• no membrane is disposed on the imaginary cross-sectional plane along the longitudinal axis of the cylinder.

10. A device according to claim 9, **characterized in that**, for input and removal of gas, every end plate of the cylinder contains at least two ports, which are respectively disposed above and below the imaginary cross-sectional plane, and so the supply chamber containing the hollow-filament membranes can be rotated around its longitudinal axis during supply of medium and gas.

11. A device according to claim 9 and 10, **characterized in that** the ports for the gas supply are mounted not on the head faces but on the cylinder shell, above and below the imaginary cross-sectional plane.

12. A device according to claim 9, **characterized in that** the hollow-filament membranes have a wall thickness smaller than 300 µm, a water permeability of greater than 6 m³/m²*h*bar and a pore diameter of 0.1 to 1 µm.

13. A device according to claim 9, **characterized in that** the membranes are disposed in a hexagonal array in the supply chamber.

14. A device according to claim 9, **characterized in that** at least one tubing port for medium perfusion and at least one inlet to the culture chamber are disposed on the head faces.

15. A device according to at least one of claims 10 to 14, **characterized in that** it additionally contains tubings, gas humidifiers, a medium trap in the gas line, an ultrafiltration unit in a product-harvesting line, a hardware unit, pumps, compressors, measuring and control units as well as a drive motor and a frame, to permit mounting and rotation of the device.

16. The use of the device according to claims 9 to 15 for cultivation of cells in high densities and for the recovery of cell products, cell constituents, viruses, proteins or low molecular weight substances.

17. The use according to claim 16 for recovery of drugs.

18. The use according to claims 16 and 17 for synthesis of diagnostic reagents.

## Revendications

1. Procédé de culture de cellules dans un bioréacteur à exposition à phases gazeuse ou liquide doté d'un espace d'alimentation dans lequel se trouvent des membranes à fibres creuses avec un diamètre intérieur ne dépassant pas 5 mm et dont le volume intérieur forme les chambres de culture, **caractérisé par** les étapes suivantes :
- Acheminement des cellules dans les chambres de culture
- Remplissage de l'espace d'alimentation avec le milieu nutritif (50 %) et un mélange gazeux (50 % restants)
- Mise en marche simultanée ou décalée de la perfusion de milieu ou de gaz
- Exposition cyclique des membranes à fibres creuses et des cellules qu'elles contiennent en phase gazeuse ou liquide

2. Procédé selon la revendication 1, **caractérisé en ce que** les membranes à fibres creuses sont positionnées horizontalement, à l'issue du remplissage du réacteur la moitié des membranes sont recouvertes par le milieu nutritif et la rotation du réacteur à 360° dans un sens puis dans l'autre sens permet d'atteindre l'exposition cyclique des membranes à fibres creuses.

3. Procédé selon la revendication 2, **caractérisé en ce que** la rotation est opérée selon deux étapes à 180°, séparées l'une de l'autre par des temps d'attente variables, si bien que chaque membrane à fibres creuses séjourne durant la même durée en phase liquide et en phase gazeuse.

4. Procédé selon la revendication 1, **caractérisé en ce que** l'exposition cyclique des membranes à fibres creuses est obtenue en les plongeant dans le milieu nutritif puis en les ressortant en phase gazeuse.

5. Procédé selon la revendication 1, **caractérisé en ce que** des cellules de faible densité sont acheminées dans l'espace de culture pour devenir des cellules à haute densité.

6. Procédé selon la revendication 5, **caractérisé en ce qu'**il est envisageable d'acheminer dans l'espace de culture les cellules de la plus faible densité cellulaire conjointement aux gels de polypeptides liés, qui présentent une part de glutamine élevée et / ou conjointement à des milieux semi-solides constitués à partir de liquides visqueux ou de liquides composés de petites entités de gel microscopiques.

7. Procédé selon la revendication 1, **caractérisé en ce que** les cellules concernées sont des protozoaires, des bactéries, des levures, des champignons, des cellules végétales ou de mammifères.

8. Procédé selon la revendication 1, **caractérisé en ce que** l'acheminement des cellules dans les chambres s'opère via un système central d'alimentation en dehors de l'espace d'alimentation, si bien qu'il est possible d'apporter simultanément [de manière homogène] les cellules dans toutes les chambres par un point de raccord.

9. Dispositif de mise en culture et de culture de cellules dans un espace d'alimentation à deux phases cylindrique ou sphérique (pouvant être alimenté par du gaz ou par le milieu), dans lequel- parallèlement à l'axe longitudinal du manteau cylindrique- des membranes à fibres creuses polymériques destinées à la micro filtration et retenant les cellules, dont le diamètre intérieur ne dépasse pas 5 mm, sont fixées aux plaques terminales, dont le volume intérieur forme les chambres de culture dans lesquelles se trouvent les cellules à cultiver, **caractérisé en ce que**
- l'espace d'alimentation comporte une phase gazeuse pouvant contenir un mélange gazeux, ainsi qu'une phase liquide pouvant contenir un milieu de culture
- chaque membrane à fibres creuses présente via la longueur du cylindre un intervalle d'au moins 0,5 mm par rapport à la membrane à fibres creuses suivante
- les membranes à fibres creuses sont positionnées de façon symétrique, par rapport à une section transversale prévue, le long de l'axe de rotation du cylindre
- et aucune membrane ne se situe sur le plan transversal prévu le long de l'axe de rotation du cylindre.

10. Dispositif selon la revendication 9, **caractérisé en ce que** chaque plaque terminale du cylindre contient au moins deux points de raccord pour l'apport et l'évacuation du gaz, se trouvant respectivement au-dessus et en-dessous du plan transversal prévu, si bien que l'espace d'alimentation avec les membranes à fibres creuses peut être porté en rotation autour de son axe longitudinal lors de l'approvisionnement avec le milieu ou du gaz.

11. Dispositif selon les revendications 9 et 10, **caractérisé en ce que** les points de raccord pour l'approvisionnement en gaz peuvent être placés au niveau du manteau cylindrique, au-dessus et en-dessous du plan transversal prévu, et non au niveau des surfaces de tête.

12. Dispositif selon la revendication 9, **caractérisé en ce que** les membranes à fibres creuses présentent une épaisseur de paroi inférieure à 300 µm, une perméabilité à l'eau supérieure à 6 m³/m²*h*bar ainsi qu'un diamètre des pores compris entre 0,1 et 1,0 µm

13. Dispositif selon la revendication 9, **caractérisé en ce que** les membranes sont positionnées de manière hexagonale dans l'espace d'alimentation.

14. Dispositif selon la revendication 9, **caractérisé en ce qu'**au moins un raccord-tuyau à la perfusion de milieu et au moins un accès à l'espace de culture se situent au niveau des surfaces de tête.

15. Dispositif selon au moins l'une des revendications 10 à 14, **caractérisé en ce qu'**il contient en outre des tuyaux, des humidificateurs de gaz, un collecteur de milieu dans la rampe à gaz, une unité d'ultrafiltration dans la rampe à produit récolté, une unité logicielle, des pompes, des unités de mesure et de réglage ainsi qu'un moteur d'entraînement et un support permettant l'entreposage et la rotation du dispositif.

16. Utilisation du dispositif selon les revendications 9 à 15, destinée à la culture de cellules de hautes densités et à l'obtention de produits cellulaires, éléments cellulaires, virus, protéines ou substances à faible poids moléculaire.

17. Utilisation selon la revendication 16 destinée à l'obtention de médicaments.

18. Utilisation selon les revendications 16 et 17 destinée à la fabrication de produits diagnostiques.
